# EUROPEAN PATENT APPLICATION

(11) **EP 4 227 724 A1**
(43) Date of publication of application: **16.08.2023**
(21) Application number: 22156635.9
(22) Date of filing: 14.02.2022
(51) Int. Cl.: G02B 21/34, G02B 21/36, G06F 3/00, C12M 1/00

(54) **MICROSCOPE PARAMETER CONTROLLER, MICROSCOPE ARRANGEMENT AND METHOD FOR CONTROLLING MICROSCOPE PARAMETERS**

(71) Applicant: Leica Microsystems CMS GmbH, 35578 Wetzlar (DE)
(72) Inventor: Christ, Stefan, 35641 Schöffengrund (DE)
(74) Representative: DehnsGermany Partnerschaft von Patentanwälten

(57) **Abstract**

The present invention relates to a microscope parameter controller (140) for controlling microscope parameters, and to a microscope arrangement (160) including such a microscope parameter controller (140), and to a method for controlling microscope parameters. A microscope parameter controller (140) for controlling microscope parameters for handling, maintaining and/or imaging a sample (120) is configured to determine at least one predefined setting of microscope parameters from one or more of user-defined user settings of microscope parameters.

## Description

### Technical field

The present invention relates to a microscope parameter controller for controlling microscope parameters, and to a microscope arrangement including such a microscope parameter controller, and to a method for controlling microscope parameters, more particularly, to the field of controlling microscope parameters for handling, maintaining and/or imaging a sample before and/or during microscopic examination.

### Background

Especially in the field of microscopic examination of living samples like cells, it is of great interest to keep the sample as long as possible under favourable and stress-free environmental conditions. To this end, incubators can be used for generating an incubation atmosphere or microclimate adapted to the sample to be examined. Microscopic examination of a sample, particularly a living sample, typically involves handling the sample, maintaining/preserving the sample during and before examination and/or imaging the sample.

Incubators can be distinguished in stage top incubators, on the one hand, and cage incubators, on the other hand. Cage incubators are, typically, mounted to a standard microscope and comprise a large climatic chamber covering the main parts of the microscope, such as the objective revolver, the microscope stage including the sample carrier and an illumination condenser, such that a large volume needs to be incubated. On the other hand, a stage top incubator provides a small volume to be incubated as the stage top incubator only encloses the sample itself and is placed onto the microscope stage. While a cage incubator entails relatively high energy and gas consumption to maintain the required incubation atmosphere, a stage top incubator provides a small closed incubated space including connected supply conduits for supplying the desired incubation atmosphere.

When examining a sample, particularly using such a microscope and incubator, various microscope parameters have to be set automatically and/or by a user in order to be able to properly handle and maintain a sample before and during examination and to choose a suited imaging mode for imaging the sample. To this end, the user needs to have a good understanding of the sample properties, on the one hand, and of the technical parameters of the microscope including the incubator.

### Summary

In view of the situation described above, there is a need for an improved control of microscope parameters for handling, maintaining and/or imaging a sample before and during examination by a microscope. According to embodiments of the invention, a microscope parameter controller, a microscope arrangement including such a microscope controller, a method for controlling microscope parameters, and a computer program for performing such a method according to the independent claims are provided. Embodiments are the subject matter of the dependent claims and of the description as follows.

Embodiments of the invention relate to a microscope parameter controller for controlling microscope parameters for handling, maintaining and/or imaging a sample, said microscope parameter controller being configured to determine at least one predefined setting of microscope parameters from one or more of user-defined user settings of microscope parameters.

"Handling" a sample comprises e.g. placing a sample onto a microscope stage of a microscope, removing the sample from the stage, and/or manipulating the sample during or before examination. "Maintaining" a sample comprises maintaining or preserving a sample before and/or during examination, particularly during imaging of the sample by means of the microscope and its accessories like a sample atmosphere incubator. "Imaging" a sample comprises illuminating the sample and imaging the sample by means of imaging optics of the microscope. Possible imaging methods comprise e.g. widefield, lightsheet and confocal microscopy including fluorescence microscopy.

"Microscope parameters for handling, maintaining and/or imaging a sample" comprise, especially in case of an incubated sample, sample incubation atmosphere parameters and microscope imaging parameters. Such parameters are, for instance, the temperature at the sample and/or of the incubation atmosphere, the relative humidity and a CO₂ and/or N₂ content of the incubation atmosphere, the incubation mode, like cage or stage top incubation or integrated sample chamber incubation as explained below, the kind of sample carrier, like Petri dish, multiwell plate, etc. Further, the imaging mode, like widefield, lightsheet or confocal microscopy, and in case of fluorescence microscopy, excitation wavelengths and emission or observation wavelengths. Further microscope parameters may be associated with illumination intensity, magnification of microscope objective and any other parameters influencing imaging, maintaining or manipulation of a sample.

"Determining at least one predefined setting of microscope parameters from one or more of user-defined user settings of microscope parameters" may generally comprise registering/remembering all or at least a part of user-defined user settings of microscope parameters, which user-defined user settings may be microscope parameter settings previously input by a user and/or otherwise used by a user. A microscope parameter setting input and/or otherwise used by a user comprises microscope parameters, which are input by a user and/or selected and/or accepted from a number of predefined microscope parameters and/or accepted microscope parameters from factory defaults (recommendations) such as e.g. certain temperature settings for certain specific samples types. Such a registering may be performed by storing the microscope parameter settings as input or otherwise used by a user in a memory/storage e.g. together with a time indication of the time of input/use of the respective user setting, and, optionally, together with a user identification (ID). The time indication may comprise a clock time and/or a sequence indication like "previous", "penultimate" etc. or "first", "second", "third" etc. usage of input/used user setting. It is noted that it is not necessary to store each and every user-defined user setting input or used by a user. In an embodiment, the microscope parameter controller is a learning system, which adapts to a user, either user-specific or in relation to a plurality of users, such that e.g. the most frequently used user setting or e.g. the two most frequently used user settings are stored while other rarely or only once used user settings are discarded. Thus, alternatively or additionally to a time indication, a probability indication may be used for designating a user setting as the most, second most etc. frequently used user setting. In a simple embodiment, only the most frequently used user setting is stored. The microscope parameter controller is further configured to process said one or more user-defined user settings as will be explained in further detail below, to determine at least one predefined setting of microscope parameters, i.e. at least one microscope parameter setting defined by the microscope parameter controller, which microscope parameter setting thus constitutes, from a user perspective, a predefined microscope parameter setting. This one or more predefined microscope parameter setting may be suggested to a user at a next time of using the microscope and/or may be used to immediately start a microscope operation at a next time of using the microscope, as will be explained below.

In an embodiment, the microscope parameter controller is further configured to select one of the at least one predefined setting of microscope parameters for starting a microscope operation applying the selected predefined setting of microscope parameters. In other words, the microscope parameter controller automatically starts operation of the microscope with a selected predefined microscope parameter setting (e.g. fulfilling minimal requirements of microscope operation or adapting the microscope and its accessories to the needs of a specific sample type or organism) in order to more quickly achieve the operating status for examining and/or for maintaining a sample. Still, while the system already starts operation in order to reach the selected predefined setting of microscope parameters, a user may be allowed to change the selected predefined setting.

In an embodiment, the microscope parameter controller is further configured to receive a user selection of one of the at least one predefined setting of microscope parameters for a microscope operation applying the user-selected predefined setting of microscope parameters. As explained above, such a user-selected predefined setting of microscope parameters can also be regarded a user-defined user setting of microscope parameters and registered accordingly, e.g. regarding a frequency of use.

In an embodiment, the microscope parameter controller is further configured to determine at least one predefined setting of microscopic parameters user-specifically for one or more users of a microscope. This increase safety of operation and enables user-specific traceability of use.

In an embodiment, the at least one predefined setting of microscope parameters is determined in dependence of the current user of the microscope. In other words, the one or more predefined settings of microscope parameters are user-specifically proposed to a user.

In an embodiment, the at least one predefined setting of microscope parameters is determined as at least one of: one or more of the previous user settings; the most frequently used user setting; the second, third or n^{th}, n>3, most frequently used user setting; and a setting comprising one or more mean values of one or more respective microscope parameters of previous user settings. This saves considerable time in defining a microscope parameter setting, especially in case of recurrent identical or similar settings.

In an embodiment, the microscope parameter controller is configured to control sample incubation atmosphere parameters of a sample incubation atmosphere provided for handling, maintaining and/or imaging of an incubated sample. In this embodiment, the microscope parameter controller can be regarded a "sample incubation atmosphere parameter controller". This and the following embodiments and their advantages will be described in further detail below.

In an embodiment, the sample incubation atmosphere parameters comprise at least one of a temperature, a carbon dioxide concentration, a humidity content of the sample incubation atmosphere, and a kind of sample carrier used for carrying the sample.

In an embodiment, the microscope parameter controller is configured to render a graphical user interface providing at least one symbol configured to display the at least one predefined setting of microscope parameters and/or providing at least one widget configured to receive a user input for setting at least one microscope parameter.

The term "widget" shall, in the understanding used herein, refer to any element of interaction rendered as a part of a graphical user interface including, but not limited to, elements configured for selection and for the display of elements or collections such as buttons (including radio buttons, check boxes, toggle switches, toggle buttons, split buttons, cycle buttons), sliders, list boxes, spinners, drop-down lists, menus (including context menus and pie menus), menu bars, tool bars (including ribbons), combo boxes, icons, tree views, grid views, elements configured for navigation such as links, tabs and scrollbars, elements for textual input such as text and combo boxes, elements for output of information such as labels, tool tips, help balloons, status bars, progress bars and information bars, and containers such as (modal) windows, dialog boxes, palettes, frames and canvas elements.

The present invention also relates to a microscope arrangement comprising a microscope and the microscope parameter controller according to embodiments of the present invention for controlling microscope parameters of the microscope.

In an embodiment, the microscope comprises a sample incubation system for providing a sample incubation atmosphere for handling, maintaining and/or imaging of an incubated sample, wherein the microscope parameter controller is configured to control sample incubation atmosphere parameters of a sample incubation atmosphere, and wherein the microscope parameter controller is preferably further configured for controlling other microscope parameters e.g. for a microscope imaging operation.

The present invention also relates to a method for controlling microscope parameters for handling, maintaining and/or imaging a sample by means of a microscope, said method comprising the step of determining at least one predefined setting of microscope parameters from one or more user-defined user settings of microscope parameters.

In an embodiment, said method comprises the further step of selecting one of the at least one predefined setting of microscope parameters for starting a microscope operation applying the selected predefined setting of microscope parameters (e.g. based on type of organism analysed).

In another embodiment, the method comprises the further step of offering a user of the microscope to choose one of the at least one predefined setting of microscope parameters for a microscope operation applying the user-selected predefined setting of microscope parameters. Such a user-selected predefined setting of microscope parameters can also be regarded a user-defined user setting of microscope parameters.

In an embodiment, the microscope parameters are or comprise sample incubation atmosphere parameters, as already discussed above, of a sample incubation atmosphere provided for handling, maintaining and/or imaging of an incubated sample.

The invention also relates to a computer program with a program code for performing the method according to embodiments of the invention as described above, when the computer program is run on a processor, particularly on a microscope parameter controller according to embodiments of the present invention as described above.

Short description of the figures
- Figure 1: schematically shows a microscope having a sample incubation system and a microscope parameter controller;
- Figure 2: illustrates a graphical user interface of the microscope parameter controller and its interrelation with a sample examined by the microscope;
- Figure 3: shows an embodiment of a graphical user interface of a microscope parameter controller for receiving user input for defining a user setting of microscope parameters (Figure 3a), and an embodiment of a corresponding graphical user interface displaying a predefined microscope parameter setting (Figure 3b);
- Figure 4: illustrates a graphical user interface of a microscope parameter controller configured to receive user input for defining user settings of microscope parameters at a first usage (Figure 4a), at a second usage (Figure 4b), and the corresponding graphical user interface displaying predefined settings of microscope parameters at a third usage (Figure 4c); and
- Figure 5: illustrates a part of a graphical user interface displaying a predefined setting of microscope parameters from a first user (Figure 5a), from a second user (Figure 5b), and for all users (Figure 5c).

### Detailed description

In the following, the figures will be described comprehensively, same reference signs relating to same or at least functionally same elements. The figures and their description are to be understood as illustrating, without loss of generality, examples of a microscope parameter controller, a microscope, and a method for controlling microscope parameters according to the present invention.

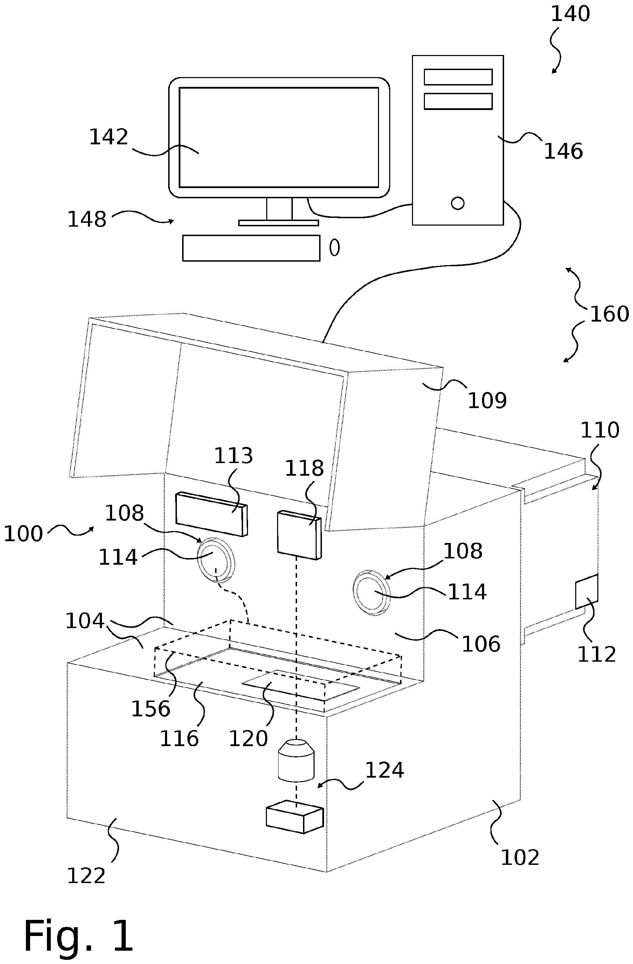

An embodiment of a microscope parameter controller is shown in Figure 1 and denoted by 140. In this embodiment, the microscope parameter controller 140 is represented by a PC 146, and renders a user interface 148; the user interface 148 comprises, e.g., a display or display screen for displaying a graphical user interface 142 (which will be further described in the figures below), and a keyboard and a computer mouse for providing user interaction. The microscope parameter controller 140 is configured to control microscope parameters for handling, maintaining and/or imaging a sample 120. "Handling" a sample 120 comprises placing a sample 120 onto a microscope stage 116 of a microscope 100 as shown in Figure 1, removing the sample 120 from the stage 116, and manipulating the sample 120 during examination. "Maintaining" a sample 120 comprises maintaining or preserving a sample 120 before and/or during examination, particularly during imaging of the sample 120 by means of the microscope 100. As already mentioned at the outset, examination of living cells, for instance, require maintaining a quite tight window of sample incubation atmosphere parameters as well as of other parameters like illumination intensity in order to avoid damaging/bleaching of the sample. "Imaging" a sample comprises illuminating the sample 120 and imaging the sample by means of imaging optics. Possible imaging methods comprise e.g. widefield, lightsheet and confocal microscopy including fluorescence microscopy.

"Microscope parameters for handling, maintaining and/or imaging a sample" thus particularly comprise, in case of an incubated sample, sample incubation atmosphere parameters and microscope imaging parameters. Such parameters are, for instance, the temperature at the sample and/or of the incubation atmosphere, the relative humidity and a CO₂ and/or N₂ content of the incubation atmosphere, the incubation mode, like cage or stage top incubation or integrated sample chamber incubation as explained below, the kind of sample carrier, like Petri dish, multiwell plate, etc. Further, the imaging mode, like widefield, lightsheet or confocal microscopy, and in case of fluorescence microscopy, excitation wavelengths and emission or observation wavelengths. Further microscope parameters may be associated with illumination intensity, magnification of microscope objective and any other parameters influencing imaging of a sample.

Through its user interface 148, the microscope parameter controller 140 is configured to receive user input setting microscope parameters for handling, maintaining and/or imaging the sample 120. "Determining at least one predefined setting of microscope parameters from one or more of user-defined user settings of microscope parameters" may generally comprise registering/remembering all or at least a part of user-defined user settings of microscope parameters, which user-defined user settings may be microscope parameter settings previously input by a user and/or otherwise used by a user, e.g. by selecting a predefined microscope parameter setting as suggested by the system. Such a registering may be performed by storing the microscope parameter settings as input or otherwise used by a user in a cache, buffer or other memory/storage e.g. together with a time indication of the time of input/use of the respective user setting, and, optionally, together with a user identification (ID). The time indication may comprise a clock time and/or a sequence indication like "previous", "penultimate" etc. or "first", "second", "third" etc. input/used user setting. It is noted that it is not necessary to store each and every user setting input or used by a user. In an embodiment, the microscope parameter controller is a learning system, which adapts to a user (or to the users) such that e.g. the most frequently used user setting or e.g. the two most frequently used user settings are stored while other rarely or only once used user settings are discarded. Thus, alternatively or additionally to a time indication, a probability indication may be used for designating a user setting as the most, second most etc. frequently used user setting. In a simple embodiment, only the most frequently used user setting is stored. The microscope parameter controller 140 is further configured to determine at least one predefined setting of microscope parameters from one or more of the tracked user-defined user settings as will be explained further below.

The controller 140 may include one or more processors. The processor(s) can be of any type and can be provided in any number and at any position and in any component of the microscope 100. As used herein, the term processor(s) may refer to any type of computational circuit, such as, but not limited to, a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), a multiple core processor, a field programmable gate array unit (FPGA). Other types of processing circuits that may be included in the controller 140 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems.

The memory/storage of the controller 140 may comprise one or more memory elements suitable to the particular application, such as a main memory in the form of random access memory (RAM), one or more hard drives, and/or one or more drives that handle removable media such as compact disks (CD), flash memory cards, digital video disk (DVD), and the like.

Before discussing the way of determining at least one predefined setting of microscope parameters by the microscope parameter controller 140 in more detail, the embodiment of microscope 100 shown in Figure 1 is now described in more detail.

Figure 1 schematically illustrates, in a perspective view, a microscope arrangement 160 comprising a microscope 100 for microscopic examination of a sample 120, and a microscope parameter controller 140 according to an embodiment of the invention. Microscope 100 comprises a sample incubation system 110. The controller 140 represented by PC 146 is connected with the user interface 148 such that a user may control microscope operation through user interface 148 via microscope parameter controller 140 which is connected to microscope 100. Note that the functionality of the PC 146 can also be performed by one or more processors as described above, and/or the controller 140 can be integrated into the microscope housing 102 and thus be part of the microscope 100. The user interface 148 can, for example, also comprise a touchscreen for receiving user input.

Sample 120 can be placed onto microscope stage 116. Microscope housing 102 encloses an illumination optics 118, the microscope stage 116 and an imaging optics 124. An integrated sample chamber 106 is located within the microscope housing 102 and formed by a separated housing section 104 within said microscope housing 102. The housing 102 comprises a hinged lid 109, which provides, in its opened state, direct access to the microscope stage 116 for placing, removing and/or manipulating the sample 120 in the sample chamber 106.

The embodiment shown in Figure 1 is an inverse transmitted-light microscope 100 where the transmitted-light illumination optics 118 is arranged within the housing section 104, while the imaging optics 124 is located below the microscope stage 116 in another housing section, forming an imaging optics chamber 122. The imaging optics 124 typically includes a microscope objective and an image detector as the main components. The image detector usually comprises a camera, which generates microscopic images, which are typically displayed on a display screen, like the display screen of user interface 148 or on a separate part of this display or on another display (not shown). It is noted again that embodiments of the present invention may also refer to other types of microscopes like upright microscopes, incident-light microscopes, confocal microscopes, widefield microscopes etc.

The construction of the microscope housing section 104 allows - after closing the lid 109 - to form a dedicated sample chamber 106, which constitutes a closed space, which can be incubated such that sample 120, like living cells, can be kept under favourable and stress-free environmental conditions. To this end, microscope 100 further comprises a sample incubation system 110. In the embodiment shown in Figure 1, sample incubation system 110 is connected to the backside of microscope housing 102. The housing section 104 comprises an opening 114 (in this embodiment two openings 114) for receiving a conduit 108, through which incubation atmosphere can be introduced into the sample chamber 106. Thus, environmental conditions in the sample chamber 106 can be controlled by the sample incubation system 110 connected to the sample chamber 106 ("sample chamber incubation"). Incubation atmosphere can be introduced through at least one of the conduits 114 into the sample chamber 106. Depending on an existing leakage of the sample chamber 106, a part of the incubation atmosphere is allowed to escape the sample chamber 106. On the other hand, a part of the incubation atmosphere can also be withdrawn from the sample chamber 106 e.g. via another one of conduits 114.

Suitable incubation atmospheres comprise, e.g., air with a predefined content of H₂O (water or water vapour, relative humidity) and a predefined content of CO₂ (carbon dioxide). It may also be desirable to conduct hypoxia experiments with a deficiency of oxygen in the atmosphere. For further details about specific parameters and suitable or preferred ranges of sample incubation atmosphere parameters, please refer to the description of the following figures.

Note that controlling the relevant parameters (incubation atmosphere parameters like temperature, humidity, carbon dioxide concentration, oxygen concentration, and other parameters like fan speed, and possibly further microscope parameters relating to imaging of a sample as explained above), in this embodiment, will be performed in an automated manner by means of the microscope parameter controller 140. To this end, target setpoints for at least a part of said parameters can be set by a user of the microscope 100, other parameters may be predefined or even fixedly predetermined. In order to control said incubation atmosphere parameters, it is preferred to arrange sensors in or at at least one of the conduits 108, the sample chamber 106 and/or the microscope stage 116 close to the sample 120.

For increasing the lifespan of the imaging optics 124, and, particularly, in case of using immersion objectives, an atmosphere control for the imaging optics chamber 122 may be provided. Such an atmosphere control may also be operated by the microscope parameter controller 140.

As already explained above, microscope parameter controller 140 is configured to control the operation of the sample incubation system 110. To this end, in this embodiment, the microscope parameter controller is connected to a sample incubation controller 112, which is controlled by microscope parameter controller 140 and provided for implementing and monitoring/checking the sample incubation atmosphere parameters as set by the microscope parameter controller 140. At the backside of the housing section 104, there is an atmosphere regulation module 113, which includes the above-mentioned sensors for detecting actual values of incubation atmosphere parameters and/or for receiving such sensor signals. Atmosphere regulation module 113 is connected to the sample incubation controller 112 of the sample incubation system 110. In that way, a feedback control can be implemented to set a group of sample incubation atmosphere parameters to the desired setpoints. It is noted that, in other embodiments, the sample incubation controller 112 can be part of or integrated into microscope parameter controller 140.

Apart from the above-described sample chamber incubation, another incubation mode, namely a stage top incubation may be realised by a stage top sample chamber 156 as indicated by dashed lines. A connection to an opening 114 and/or a conduit 108 can also be established in order to provide the respective incubation atmosphere into the smaller stage top sample chamber 156. As explained further below, the user can select which incubation mode to use, in this embodiment, either sample chamber incubation or stage top incubation.

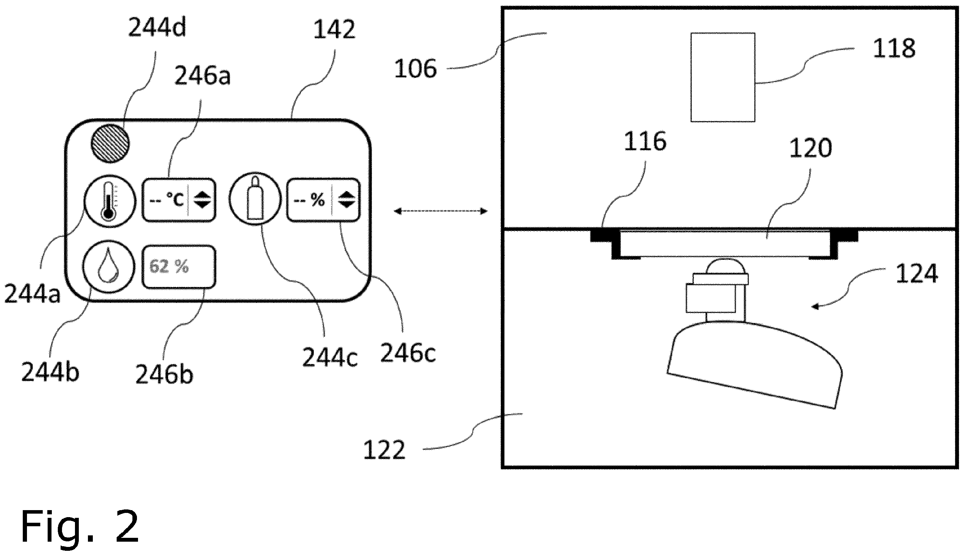

Figure 2 schematically illustrates the interrelation between the microscope parameter controller 140, namely the graphical user interface 142, and the sample 120 to be examined by the microscope 100 of Figure 1. The embodiment of a graphical user interface 142 according to Figure 2 relates to a subset of possible microscope parameters as explained in detail above. Without loss of generality, in the following, mainly sample incubation atmosphere parameters are discussed. This discussion is valid for any other microscope parameters as mentioned above in an analogous manner.

Graphical user interface 142 of the microscope parameter controller 140 displays various symbols 244a, 244b, and 244c for parameters, which are, at least in part, adjustable by corresponding widgets 246a, 246b, and 246c. In the embodiment shown, 244a is a symbol for the temperature of the sample or of the sample incubation atmosphere, 244b is a symbol for the relative humidity of the incubation atmosphere, and 244c is a symbol for the carbon dioxide (CO₂) concentration in the sample chamber 106. Said parameters may be set either by a user or may be predefined by the microscope parameter controller 140, optionally including the option of a user altering a predefined parameter. In the embodiment shown in Figure 2, widgets are provided for receiving a user input for setting/adjusting the parameters, particularly for setting target setpoints for said parameters. Note that other means of receiving a user input for setting/adjusting parameters are possible. Widget 246a receives a user input for rising or lowering a target setpoint for the temperature, for instance, via clicking on respective soft buttons. Widget 246b shows a (predefined) value of relative humidity, which value may be altered by user input as explained further below. Widget 246c provides the user the possibility of rising or lowering the target setpoint for the CO₂ concentration via clicking on respective soft buttons of widget 246c. It is noted that it is not necessary for all incubation atmosphere parameters to be changeable by a user; for instance, the relative humidity may be automatically set by the microscope parameter controller 140 and the corresponding target setpoint may only be displayed for information purposes to a user via widget 246b. The graphical user interface 142 further displays a coloured symbol 244d, representing a (virtual) lamp. Such lamp may switch from red to green when the sample examination can start, for example, when all the target setpoints are reached or when at least a number of parameters have reached their target setpoints or when at least a predefined percentage (threshold) of a target setpoint has been reached. Examples will be explained in more detail further below.

The right side of Figure 2 illustrates the sample chamber 106 and the imaging optics chamber 122 in a very schematic way. In particular, illumination optics 118, the microscope stage 116, the sample 120, and the imaging optics 124 are shown. Note that the imaging optics 124, by means of example, comprises a motorized (immersion) objective on a turret. In this and the following embodiments, only a sample chamber incubation of sample chamber 106 is discussed. The discussion is also valid for other incubation modes, like stage top incubation of stage top chamber 156 or incubation of the imaging optics chamber 122 (see also the explanations given above in connection with Figure 1).

By user input via widgets 246a, 246c and optionally 246b, target setpoints of sample incubation atmosphere parameters, namely temperature, CO₂ concentration and optionally relative humidity can be input by a user. The microscope parameter controller 140 with the support of the sample incubation controller 112 controls the sample incubation system 110 to generate and maintain the desired incubation atmosphere inside the sample chamber 106 as explained above in connection with Figure 1.

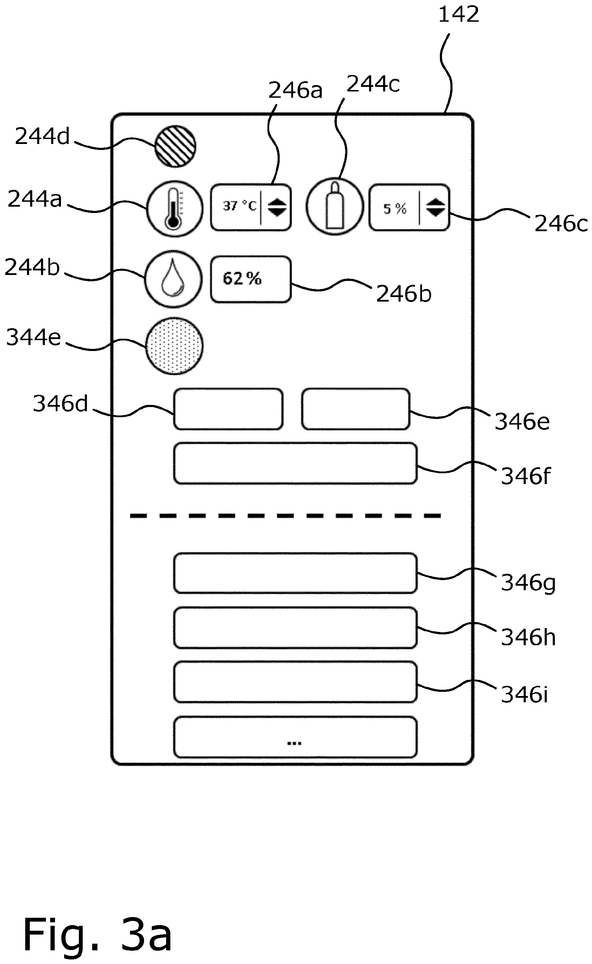

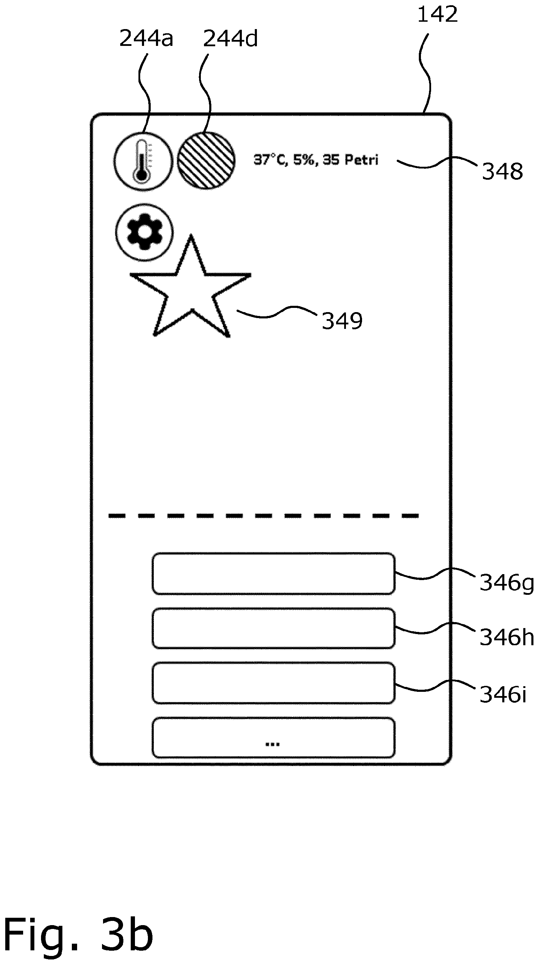

Figure 3 schematically shows another embodiment of a graphical user interface 142 of a microscope parameter controller 140 configured for receiving user input (Figure 3a), said microscope parameter controller 140 being configured to track one or more user settings of microscope parameters and to determine at least one predefined setting of microscope parameters from one or more of the tracked user settings as shown in Figure 3b.

Figure 3a shows an embodiment of a graphical user interface 142 comprising symbols 244a, 244b, 244c, and 244d and corresponding widgets 246a, 246b, and 246c as already described in connection with Figure 2. By user input, the target setpoint of the temperature is set to 37° C, the target setpoint of the CO₂ concentration is set to 5% and the predefined target setpoint of relative humidity is 62%. In this embodiment, this target setpoint can be changed by widget 346f, for example in order to reduce humidity. Further, the user can select sample chamber incubation by widget 346d or stage top incubation by widget 346e. Symbol 344e signals whether sensor calibration is still ongoing or completed.

Other widgets may be provided in graphical user interface 142 configured to receive user settings of other microscope parameters. For example, a user may select the kind of sample carrier by widget 346g, the kind of microscopic imaging mode by widget 346h, the fluorescence channel for observing a sample image by widget 346i, etc.

The microscope parameter controller 140 of this embodiment is configured to register a user setting of microscope parameters represented by widgets 246a to 246c, and 346d to 346i. Such a registering may comprise storing the user setting of microscope parameters in a memory designated to this purpose of the microscope parameter controller 140, and keeping it e.g. together with a suitable time and/or probability indication, as explained above, even if the respective user has completed sample examination and, possibly, turned off the microscope parameter controller 140. The microscope parameter controller is further configured to determine at least one predefined setting of microscope parameters from the user-defined user setting(s) such that the graphical user interface 142 of the microscope parameter controller 140 may display such a predefined setting according to Figure 3b.

As shown in Figure 3b, the graphical user interface 142 provides a symbol 348 displaying the predefined setting of microscope parameters, which is, in this example, the very setting of Figure 3a relating to one or more, preferably all of widgets 246a to 246c, and 346d to 346i. The predefined setting of microscope parameters as represented by symbol 348 is provided to the user at the next usage of microscope 100 for sample examination. For user flexibility, a widget 349 is provided for enabling the user to switch back to all settings shown in Figure 3a by e.g. clicking on widget 349.

In an embodiment, the microscope parameter controller 140 is further configured to select the predefined setting of microscope parameters as represented by widget 348 for starting a microscope operation applying the selected predefined setting of microscope parameters. In other words, the microscope parameter controller 140 automatically starts microscope 100 with microscope parameters as defined in Figure 3a in order to more quickly achieve the operating status for examining a sample, which is signalled by symbol 244d. Still, while the system already starts operation in order to reach the predefined setting of microscope parameters, a user may wish to change the predefined setting by selecting widget 349.

The inventors found that a specific user, and sometimes even a plurality of users using the same microscope 100, typically chooses the same or at least essentially the same microscope parameters, at least when examining the same kind of sample. Thus, displaying the predefined setting of microscope parameters in the form of symbol 348 to a user saves the user from again defining setpoints of microscope parameters as illustrated in Figure 3a. This saves considerable time for any user, particularly for an unexperienced user. A further timesaving is achieved by automatically starting the system immediately applying the predefined setting of microscope parameters. Another advantage of this embodiment of the invention is that a user can define the sample incubation atmosphere parameters together with other microscope imaging parameters by means of a single graphical user interface 142 combining the corresponding symbols and widgets.

Embodiments of the present invention thus, in a particular example, allow a user to examine zebrafish in Petri dishes at 28°C and a predetermined humidity and CO₂ concentration initially in transmitted light (corresponding to a first predefined microscope parameter setting), and subsequently in confocal fluorescence microscope imaging (corresponding to a second predefined microscope parameter setting) using a microscope being able to provide the corresponding imaging modes, since the microscope parameter controller 140 is able of self-learning these two predominantly used microscope parameter settings.

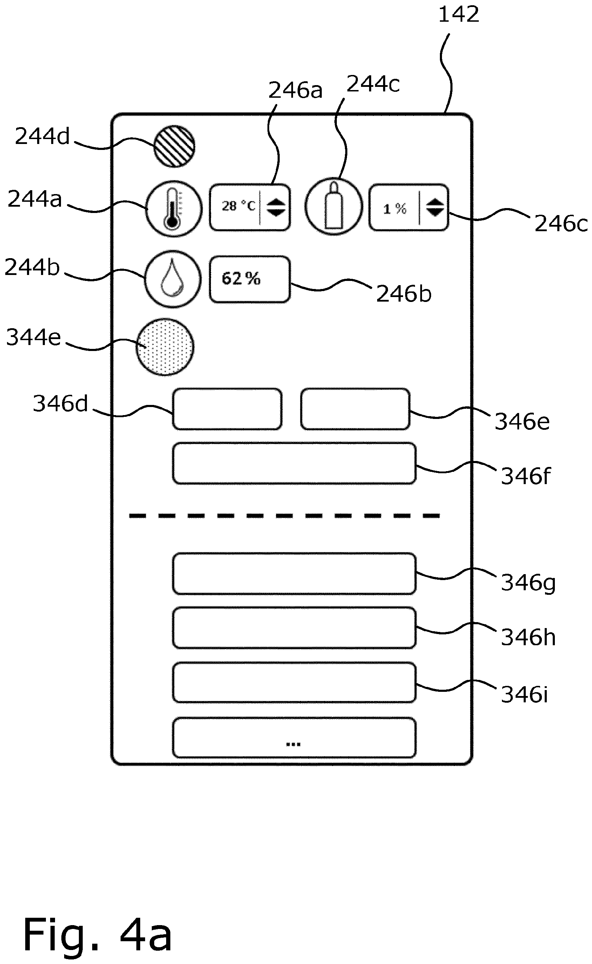

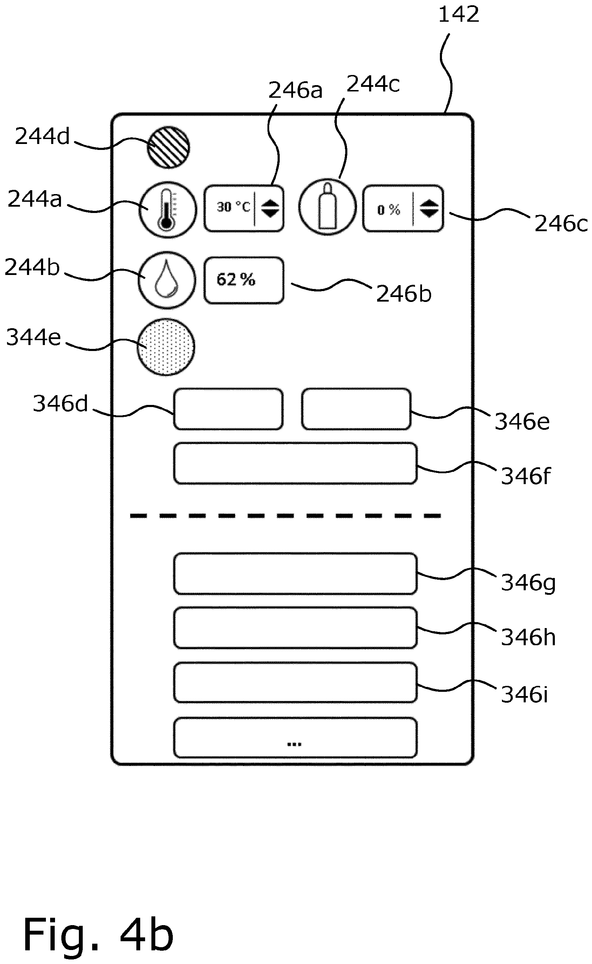

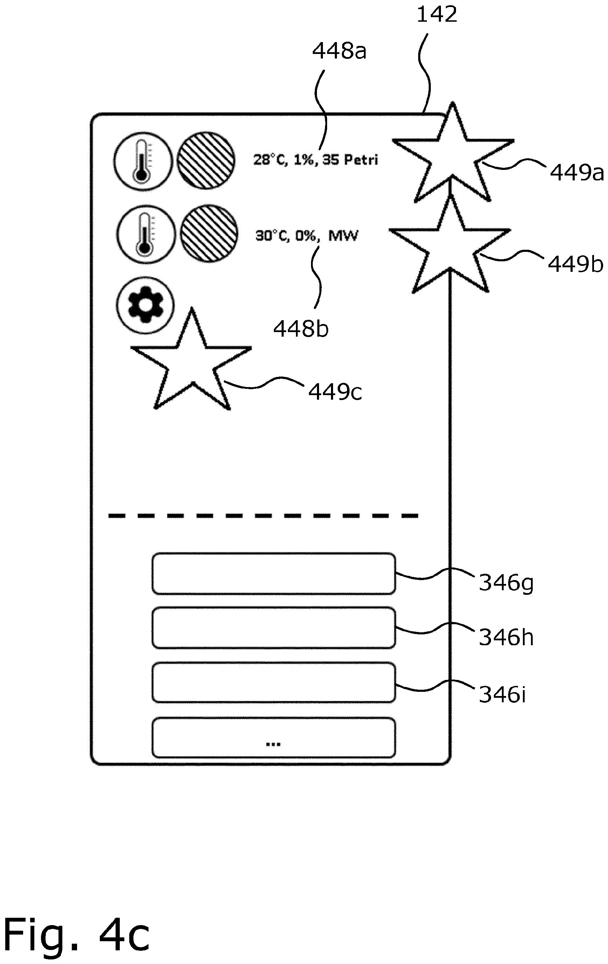

Figure 4 schematically shows an embodiment of a graphical user interface 142 at three different times of usage. Figure 4a shows the graphical user interface 142 at a first time of usage of microscope 100, Figure 4b shows the graphical user interface 142 at another/second time of usage of microscope 100, and Figure 4c shows the graphical user interface 142 at a third time of usage. The graphical user interface 142 of Figures 4a and 4b essentially corresponds to the one shown in Figure 3a. Thus, reference is made to the discussion of Figure 3a regarding symbols and widgets of the graphical user interface 142 of Figures 4a and 4b. As can be seen from Figure 4a, the user, at the first usage, has set the temperature to a setpoint of 28° C, the humidity to the standard value of 62% and the CO₂ concentration to 1% by operating widgets 246a to 246c accordingly. He/she has further selected a Petri dish as sample carrier. At the second usage, as shown in Figure 4b, the user has adjusted the setting of the first usage, particularly by not accepting the predefined (previous) setting of microscope parameters and by inputting a different setting, namely a temperature setpoint of 30° C, a relative humidity of still 62%, and a CO₂ concentration of now 0%. Further, the user has selected a multiwell plate as sample carrier. The microscope parameter controller 140 registers the user settings as shown in Figure 4a and 4b, i.e. stores the relevant set of parameters, optionally including a time and/or probability indication for further use at another time of usage of microscope 100.

This situation is illustrated by Figure 4c. The microscope parameter controller 140 determines at least one predefined setting of microscope parameters from the registered user settings at the first and the second time of usage. In this embodiment, both previous user-defined user settings are determined as the two predefined settings of microscope parameters, which are offered to the user at the third usage of the system. The two predefined settings of microscope parameters may also correspond to the two most frequently used user-defined parameter settings. Symbol 448a illustrates relevant parameters of the first predefined setting corresponding to the setting as shown in Figure 4a. Symbol 448b shows relevant parameters of the second predefined setting corresponding to the user setting as shown in Figure 4b. The microscope parameter controller 140 is further configured by means of widgets 449a and 449b to receive a user selection of one of the two predefined settings of microscope parameters for a microscope operation at the third usage by applying the selected predefined setting of microscope parameters to the operation of microscope 100. By selecting widget 449a, the user may select the parameter setting of Figure 4a, by selecting widget 449b, the user may select the parameter setting of Figure 4b. Further, another widget 449c is provided for returning to a menu where all parameter settings may be changed in order to create a third predefined parameter setting.

It is noted that a user identity may not be required for the embodiment according to Figures 4a to 4c. It may be as well that two different users input the parameter settings according to Figures 4a and 4b, while one of these users or even another third user is prompted to select or to change predefined settings as shown in Figure 4c.

In an embodiment, it is, however, possible to register user-defined user settings of microscopic parameters user-specifically for one or more users of the microscope 100. The user-specific tracking may be performed by the microscope parameter controller 140 by using, for instance, a user ID, which the user uses when logging on to the computer/PC 146 as shown in Figure 1. A user ID may also be recognised through near-field communication (NFC) by communicating with a user's ID tag or smartphone etc. In this embodiment, operation of microscope 100 may be set up to user-specifically determine one or more predefined settings of microscope parameters in dependence of the respective user of the microscope 100.

In the embodiments of Figures 3 and 4, the at least one predefined setting of microscopic parameters is determined as the previous user setting (Figure 3) or as the previous and the penultimate user settings (Figure 4). On the other hand, the microscope parameter controller 140 may determine a predefined setting of microscope parameters as the most frequently used user setting (through a number of users or regarding a specific user). Alternatively or additionally, the microscope parameter controller may determine the at least one predefined setting of microscope parameters as the second, third or, more generally, n^{th}, n>3, most frequently used user setting. Especially if all users are taken into account, it may make sense to determine the three most frequently used user settings as the predefined parameter settings displayed on the graphical user interface 142. In another embodiment, the predefined setting of microscope parameters (or one of the predefined settings of microscope parameters) comprises mean values of one or more respective microscope parameters of previous user settings. An automated system start using such a predefined parameter setting can save time to reach the desired setpoints.

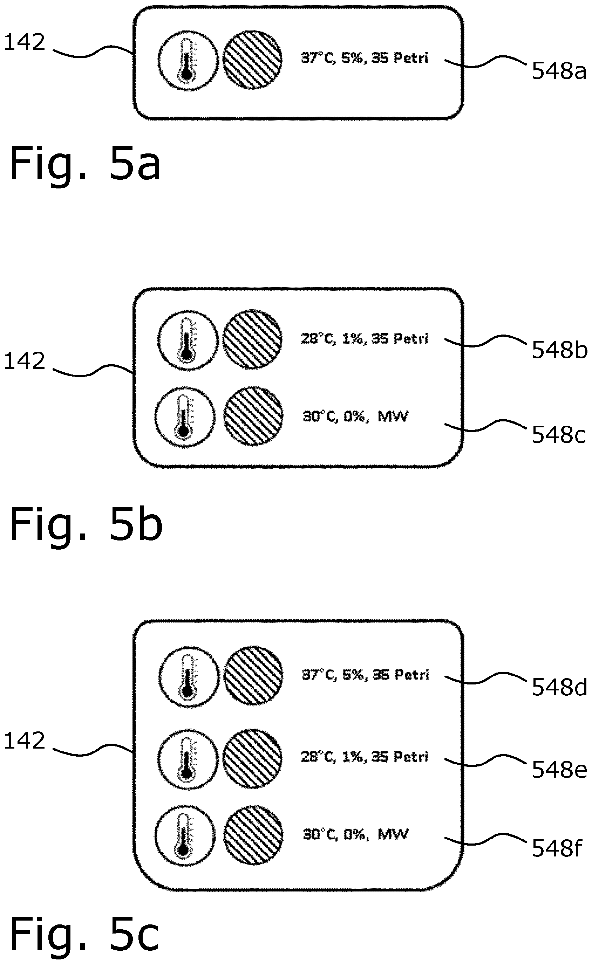

Figure 5 shows relevant parts of graphical user interfaces 142 displaying predefined settings of microscope parameters. Figure 5a shows symbol 548a representing a predefined setting of microscope parameters as personal settings of a first user. This personal setting may be the most frequent setting of this user or simply the previous setting by this user. Figure 5b shows symbols 548b and 548c representing two different predefined settings of microscope parameters as an example of personal settings by another second user. Again, the predefined parameter settings may include a most frequent user setting and/or a previous user setting. It is noted that other possibilities of determining a predefined parameter setting were discussed above.

Figure 5c shows symbols 548d, 548e, and 548f representing three different predefined parameter settings as an example of all user frequent settings. The respective predefined parameter settings may represent the three most frequently used user settings throughout the users. Other possibilities of determining predefined parameter settings have been discussed above.

As used herein the term "and/or" includes any and all combinations of one or more of the associated listed items and may be abbreviated as "/".

Although some aspects have been described in the context of an apparatus, it is clear that these aspects also represent a description of the corresponding method. Analogously, aspects described in the context of a method step also represent a description of a corresponding feature of a corresponding apparatus.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may e.g. comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of Reference Signs

- 100: microscope
- 102: microscope housing
- 104: separated housing section
- 106: sample chamber
- 108: conduit
- 109: lid
- 110: sample incubation system
- 112: sample incubation controller
- 113: atmosphere regulation module
- 114: opening
- 116: microscope stage
- 118: illumination optics
- 120: sample
- 122: imaging optics chamber
- 124: imaging optics
- 140: microscope parameter controller
- 142: graphical user interface
- 146: PC
- 148: user interface
- 156: stage top chamber
- 160: microscope arrangement
- 244 a, b, c, d: symbol
- 246 a, b, c: widget
- 344 e: symbol
- 346 d-i: widget
- 348: symbol
- 448 a, b: symbol
- 449 a, b, c: widget
- 548 a-f: symbol

## Claims

1. A microscope parameter controller (140) for controlling microscope parameters for handling, maintaining and/or imaging a sample (120), said microscope parameter controller (140) being configured to determine at least one predefined setting of microscope parameters from one or more of user-defined user settings of microscope parameters.

2. The microscope parameter controller (140) according to claim 1, wherein the microscope parameter controller (140) is further configured to select one of the at least one predefined setting of microscope parameters for starting a microscope operation applying the selected predefined setting of microscope parameters.

3. The microscope parameter controller (140) according to claim 1 or 2, wherein the microscope parameter controller (140) is further configured to receive a user selection of one of the at least one predefined setting of microscope parameters for a microscope operation applying the user-selected predefined setting of microscope parameters.

4. The microscope parameter controller (140) according to any one of the preceding claims, wherein the microscope parameter controller (140) is further configured to determine at least one predefined setting of microscope parameters user-specifically for one or more users of a microscope (100).

5. The microscope parameter controller (140) according to any one of the preceding claims, wherein the at least one predefined setting of microscope parameters is determined as at least one of:
the previous user setting or previous user settings;
the most frequently used user setting;
the second, third or n^{th}, n>3, most frequently used user setting; and
a setting comprising mean values of one or more respective microscope parameters of previous user settings.

6. The microscope parameter controller (140) according to any one of the preceding claims, wherein the microscope parameter controller (140) is configured to control sample incubation atmosphere parameters of a sample incubation atmosphere provided for handling, maintaining and/or imaging of an incubated sample (120).

7. The microscope parameter controller (140) according to claim 6, wherein the sample incubation atmosphere parameters comprise at least one of a temperature, a carbon dioxide concentration, a humidity content of the sample incubation atmosphere, and a kind of sample carrier used for carrying the sample (120).

8. The microscope parameter controller (140) according to any one of the preceding claims, wherein the microscope parameter controller (140) is configured to render a graphical user interface (142) providing at least one symbol (348, 448a-b, 548a-f) configured to display the at least one predefined setting of microscope parameters and/or providing at least one widget (246a-c, 346d-j, 349, 449 a-c) configured to receive a user input for setting one or more microscope parameters.

9. A microscope arrangement (160) comprising a microscope (100) and the microscope parameter controller (140) of any one of the preceding claims for controlling microscope parameters of the microscope (100).

10. The microscope arrangement (160) according to claim 9, wherein the microscope (100) comprises a sample incubation system (110) for providing a sample incubation atmosphere for handling, maintaining and/or imaging of an incubated sample (120), wherein the microscope parameter controller (140) is configured to control sample incubation atmosphere parameters of the sample incubation atmosphere, and wherein the microscope parameter controller (140) is preferably further configured for controlling a microscope imaging operation.

11. A method for controlling microscope parameters for handling, maintaining and/or imaging a sample (120) by means of a microscope (100), said method comprising the step of
determining at least one predefined setting of microscope parameters from one or more user defined user settings of microscope parameters.

12. The method for controlling microscope parameters of claim 11, said method comprising the further step of
selecting one of the at least one predefined setting of microscope parameters for starting a microscope operation applying the selected predefined setting of microscope parameters.

13. The method for controlling microscope parameters of claim 11 or 12, said method comprising the further step of
offering a user of the microscope (100) to choose one of the at least one predefined setting of microscope parameters for a microscope operation applying the user-selected predefined setting of microscope parameters.

14. The method for controlling microscope parameters of any one of claims 11 to 13, the microscope parameters being or comprising sample incubation atmosphere parameters of a sample incubation atmosphere provided for handling, maintaining and/or imaging of an incubated sample (120).

15. Computer program with a program code for performing the method according to any one of claims 11 to 14, when the computer program is run on a processor, particularly on a microscope parameter controller (140) according to any one of claims 1 to 8.
